# EUROPEAN PATENT SPECIFICATION

(11) **EP 0 941 734 B1**
(45) Date of publication and mention of the grant of the patent: **20.11.2002**
(21) Application number: 99104298.7
(22) Date of filing: 03.03.1999
(51) Int. Cl.: A61K 35/08, A61P 3/00

(54) **Artificial mineral water for medicinal/prophylatic purposes**
Künstliches Mineralwasser für medizinische/prophylaktische Zwecke
Eau minérale artificielle à usage médical/prophylactique

(30) Priority: 04.03.1998 LV 980035
(43) Date of publication of application: 15.09.1999
(73) Proprietor: Ponomarenko, Elena, 1082 Riga (LV)
(72) Inventor: Ponomarenko, Elena, 1082 Riga (LV)
(74) Representative: Madgwick, Paul Roland

(56) References cited:
- GB-A- 2 107 184
- DATABASE WPI Section Ch, Week 9832 Derwent Publications Ltd., London, GB; Class B06, AN 98-375522 XP002104106 & RU 2 099 062 C (ASGOR RES LAB) , 20 December 1997
- DATABASE WPI Section Ch, Week 9741 Derwent Publications Ltd., London, GB; Class B06, AN 97-446910 XP002104107 & RU 2 074 723 C (MASHEKOLOGIYA RES TECH CENTRE), 10 March 1997

## Description

The present invention concerns artificial medicinal-prophylactic mineral water.

### STATE OF THE ART

Even with considerable progress in pharmacology and medicinal chemistry, resulting in design of new medicines with either specific or wide-spectrum activity, there is a substantial interest in "non-pharmaceutical" methods of treatment namely treatment by means of mineral waters.

Medicinal mineral waters are natural waters, containing mineral (rarely also organic) components. Depending on the concentration of minerals such waters are divided into waters of:
1) slight mineralization (0.3 - 2.0 g/l);
2) low mineralization (2.0 - 5.0 g/l);
3) medium mineralization (5.0-15.0 g/l);
4) high mineralization (15.0 - 35.0 g/l).

An example of a slightly mineralized water is water from Naftusia spring in Truskovets spa. This water has attracted interest due its curative activity in disturbances of liver, bile and kidney functions. The constitution of slightly mineralized Naftusia water varies within considerable limits depending on season, water output and weather conditions. The chemical analysis of Naftusia water yielded the following averages for seven main components (g/l): Mg²⁺ - 0.045; Ca²⁺ - 0.124; (Na⁺ + K⁺) - 0.021; HCO₃⁻ - 0.476; SO₄²⁻ - 0.117; Cl⁻ - 0.021.

It has been established that the artificial analog of Naftusia water known as "ISAN", (containing in g/l - CaCO₃ - 0.21; MgCO₃ - 0.106; NaHCO₃ - 0.021; KHCO₃ - 0.018; MgSO₄ - 0.141; CaCl₂ - 0.055, with pH = 7.3 - 7.4) performs some curative functions of the natural mineral water.

Mineral waters are widely consumed by healthy people. Unfortunately the action of Naftusia water and other slightly mineralized waters on healthy persons has not been established. Natural Naftusia mineral water is unstable and cannot be stored and shipped. The artificial analog of Naftusia water has not been used for medicinal purposes [M.S. Yaremenko et al., Physiological basis of curative properties of Naftusia water, 1989 (in Russian)].

### SHORT DESCRIPTION OF THE INVENTION

The main drawback of the natural mineral water of Naftusia type is its irregular constitution and instability in storage, caused by organic components present in water. This instability excludes the shipment of the natural water over longer distances. The present invention is aimed at creating an artificial, slightly mineralized water, of constant constitution, stable in storage and shipment and capable of performing the main curative functions of the natural slightly mineralized water. The disclosed artificial medicinal water (AMW) with curative and prophylactic properties is novel.

Surprisingly we have found that a slightly mineralized water, containing in per cent of weight:

| | |
|---|---|
| magnesium sulphate | 0.0005- 0.01 |
| potassium chloride | 0.001 - 0.02 |
| tris-(hydroxymethyl)aminomethane | 0.005 - 0.02 |
| tris-(hydroxymethyl)aminomethane hydrochloride | 0.01 - 0.04 |
| distilled water | the rest up to 100, |

optionally containing also the usual soft drink additives, is efficient in treatment and prevention of certain disturbances, in particular, disturbances of kidney and liver functions, gallbladder diskinesias, diabetes mellitus, disturbances of arterial pressure, atherosclerosis changes in blood vessels, and for normalization of immunity factors of cells. Moreover, the disclosed composition is effective in the optimization of homeostasis parameters and the normalization of aberrations from homeostasis, in intensifying the excretion of radioactive metal ions, particularly Cs-137.

The advantages of the disclosed composition of the slightly mineralized AMW over the natural mineral waters, particularly Naftusia type water, are the following:
1) constant composition, in particular a stable pH, secured by addition of tris-buffer;
2) feasibility to produce the composition of this invention at essentially any location, eliminating the necessity of long-distance shipment of mineral water;
3) extended range of curative applications and use by normal healthy persons.

The disclosed AMW is convenient in use. AMW is consumed 30 - 40 minutes before meals in quantities from 200 to 400 ml 2 to 3 times daily. The curative course is 21 to 28 days. If necessary, the course may be repeated.

The disclosed AMW was tested in clinic on practically healthy persons of 16 to 60 years of age. The analysis of subjective complaints associated with treatment - weakness, shivers, itching, vertigo, diarrhoea - did not disclose any objective disturbances directly attributable to consuming of the water. The analysis of objective parameters for each nosology showed normalization of the essential clinical parameters after use of AMW. The analysis of objective parameters for internal organs of the healthy persons after treatment with AMW disclosed optimization thereof when they were in the normal range and normalization when some parameters were outside the optimal range.

Treatment of patients with disturbances of health disclosed substantial improvement of status for a number of pathologies. Thus, treatment for one day of patients with various forms of glomerulonephritis with 200 - 400 ml of water 2 to 3 times 30 to 40 minutes before meals, diminished the excreted protein in urine by 52% (Tables 1 - 4). The use of AMW not only diminished proteinuria, but also positively influenced the parameters of the kidney filtering function that was analyzed on the 4^{th} day after a single day treatment with AMW. In particular, a significant lowering of protein level in urine and significant increase of protein level in blood was determined. There was a significant lowering of cholesterol level in blood. There was a tendency for lowering of blood urea, creatinine and K⁺. When patients with chronic pelonephritis and nephrolitiasis were treated with AMW for 7 days, a significant lowering of leukocytes and erythrocytes in urine, lowering of proteinuria, increased diuresis (daily average increase of 966 ml) was established. After use of AMW the patients displayed normalized leukocytary formula of blood, normalized erythrocyte sedimentation rate (Tables 5 - 10). Patients with nephrolitiasis excreted with urea minute stones.

For hypertensive patients lowering of arterial pressure was observed. For patients with virus A hepatitis after a 3 day treatment with AMW lowering of blood billirubin (average decrease 42%) and lowering of AlanineAT (2 to 4 times) was observed (Tables 11 and 12). In the course of treatment by AMW of patients with chronic cholecystitis, patients with bile sludge and of normal healthy persons, it was discovered that in 30 minutes after intake of AMW the gallbladder contracted by 5 mm average, later regaining the original size. Thus intake of AMW results in increased motor activity of gallbladder, excretion of sludge - high density bile, which is a risk factor in development of sand, gallstones and cancer of the gallbladder (Tables 13 - 16).

For patients with angina pectoris, associated with arterial hypertension, a highly significant change in blood parameters were observed after 7 days of AMW treatment:
1) significant (P < 0.05) increase of HDL-cholesterol;
2) significant (P < 0.01) lowering of LDL-cholesterol;
3) significant (P < 0.02) lowering of Klimov's index;
4) average lowering of arterial pressure for 20 mm Hg (Tables 17 and 18).

For patients with diabetes mellitus after a 7 day use of AMW the normalization of glycolysed fibrinogen level in the blood was observed with attendant lowering of urine protein level by 83% average (Tables 19 and 20). For patients with immunodeficiency, 30 minutes after the intake of AMW the level of CD₃, CD₄ and CD₈ in the blood was normalized. In particular the quantity of CD₃ was increased 2.8 times, CD₄ - 3.6 times and CD₈ - 4 0 times (average numbers). The number of natural killers was increased 5 times. This effect was observable for 191 hours (Table 21).

The status of the practically healthy persons was characterized by the registering of 19 parameters, which demonstrate the status of internal organs and which are widely used in clinical practice as test indicators. reflecting protein metabolism, nitrogen balance and the components thereof, enzyme activity, carbohydrate, pigment and mineral metabolism. No drift of the aforementioned parameters outside the initial normal range and normalization of deviated parameters was registered after intake of various doses of AMW (100 - 200- 400 ml).

The intake of AMW by practically healthy persons, who have stayed for some time in a contaminated locality, resulted in an increased excretion of Cs-137. The content of Cs-137 in daily urine after a 1 day intake of AMW was found to have increased by 40% , after 10 a day treatment the level of Cs-137 in the daily urine was found to have lowered by 60% (Table 22).

### A METHOD FOR PRODUCING AMW

To produce AMW, 0.0005 g magnesium sulphate was added to 100 g of distilled water and brought into solution. 0.001 g of potassium chloride, 0.005 g tris-(hydroxymethyl)aminomethane and 0.010 g of tris-(hydroxymethyl)-aminomethane hydrochloride were added thereto. The pH of the solution was checked. The AMW thus obtained is ready for use. AMW can be stored in closed containers at temperatures from 0 °C to 18 °C for one year without deterioration of its curative properties.

### EXAMPLES OF THE APPLICATION OF AMW

### Treatment of various forms of glomerulonephritis.

21 patients were treated in a double-blind experiment. 15 out of 21 patients received AMW, 6 - placebo. The level of protein in the daily urine was determined. The results are given in Tables 1 and 2.

### Example 1.

Patient I.B. was admitted with back-aches, high arterial pressure (up to 200/100 mm Hg), proteinuria 4.84 g/l. Before treatment with pharmaceutical preparations the patient was treated with AMW for one day (200 ml 3 times 30 - 40 minutes before meals). On the following day urine was collected. The urine protein level was found to be 0.91 g/l, i.e., a lowering for 80% from the initial level was observed. Arterial pressure was 150/90 mm Hg.

Additionally in an open experiment 6 patients with proteinuric proliferative glomerulonephritis (protein in urine 6 g/l daily) and functional kidneys were given AMW for one day (2 - 3 times 200 -400 ml of AMW) and examined before and after intake of AMW. The parameters, characterizing the filtering function of the kidneys were determined 96 hours after the one day intake of AMW. The results are given in Tables 3 and 4.

### Example 2

Patient A.M. was admitted with complaints of deterioration of condition, back-aches, lowered diuresis. Before treatment with pharmaceutical preparations the patient was treated with AMW for one day. After the one day the concentration of K⁺ in the blood plasma dropped from 6.2 to 4.9 mmol/l. The concentration of Na⁺ in the blood plasma was retained at the original level. The protein concentration in the blood plasma increased from 66 to 74 g/l, the concentration of protein in the urine decreased from 8.8 to 2.6 g/l. The concentration of creatinine in the blood decreased from 1.7 to 1.4 mmol/l, while the concentration in the urine increased from 0.31 to 1.06 mmol/l. The concentration of Na⁺ in the urine also increased from 135 to 240 mmol/l. The diuresis increased by 900 ml. These results show that kidney filtering function was improved for 96 hours after one day intake of AMW. The intake of placebo did not yield comparable results.

### Chronic pelonephritis, nephrolitiasis

12 patients with acute pelonephritis and 12 patients with nephrolitiasis resulting from pelonephritis were treated in a double-blind experiment. 6 out of each group of 12 patients received AMW, 6, the controls received a placebo. AMW was taken for 7 days, 3 - 2 times daily at 200 - 400 ml dose. All patients before and after treatment were checked for proteinuria, leukocituria, erythrocytes in urine, daily diuresis, blood formula. The results are given in Tables 5 - 10.

### Example 3

Patient T.B. was admitted with high arterial pressure, erythrocyturia, burning sensation in urethra. For 7 days AMW was used as the single remedy. After 7 days analyses demonstrated decrease of erythrocytes in the urine by 80%, leukocytes by 70%. Arterial pressure dropped from 150/100 to 120/80 mm Hg. The burning sensation disappeared. Thus the effectivity of AMW in pelonephritis was established. The intake of placebo did not yield comparable results.

### Virus A hepatitis

12 patients were treated in a double-blind experiment. 6 out of 12 patients received AMW, 6 - placebo. The treatment lasted for 3 days, the daily intake was 200 - 400 ml 3 - 2 times daily. All patients were examined for billirubin level in blood, AlanineAT activity. The results are given in Tables 11 and 12.

### Example 4

Patient was admitted with weakness, jaundice, coloured urine and faeces, pyrexia. The treatment with AMW lasted for 3 days. After 3 days the level of billirubin in blood decreased from 47 mmol/l to 23 mmol/l. The AlanineAT activity decreased from 1260 units/L to 560 units/L. The body temperature reached normal level. While AMW was effective in the restoring of the basic clinical parameters, the placebo did not yield comparable results.

### Chronic cholecystitis, sludge in the gallbladder

12 patients were treated in a double-blind experiment. One dose of 400 ml AMW was given. Ultrasonography of the gallbladder for all patients was conducted. The diameter of the gallbladder was measured in a-p direction before and after the intake of the AMW or placebo. The results are given in Tables 13 - 16.

### Example 5

Patient I.S. was admitted with pains in gallbladder, eructation, bitter taste in mouth. Ultrasonographic examination demonstrated high density bile sludge in the gallbladder. After a single intake of AMW the gallbladder contracted by 5 mm, the gallbladder did not contain high density sludge, the patient no longer complained about eructation or bitter taste in mouth.

The effectiveness of AMW in increasing motor activity of the gallbladder was demonstrated, the placebo did not yield comparable results.

### Ischemic heart disease, atherosclerosis changes in blood vessels

12 patients were treated in a double-blind experiment. 6 out of 12 patients received AMW, 6 - placebo. The treatment lasted for 7 days, the dosage was 200 - 400 ml. All patients were examined for cholesterol, triglycerids, phospholipids, HDL- and LDL-cholesterol level in blood serum. Klimov's index was calculated. The results are given in Tables 17 and 18.

### Example 6

Patient A.S. was admitted with a developed ischemic disease and essential arterial hypertonic disease. After taking AMW for 7 days arterial pressure dropped from 220/11 mm Hg to 150/90 mm Hg. Subjective condition improved. Blood analysis revealed the increase of HDL-cholesterol level from 0.75 to 1.05 mmol/l and a decrease of LDL-cholesterol level from 3.97 to 2.87 mmol/l, a decrease of Klimov's index from 6.56 to 3.01. These data demonstrate a substantial decrease of atherosclerosis disturbances, the placebo did not yield comparable results.

### Diabetes mellitus

12 patients were treated in a double-blind experiment. 6 out of 12 patients received AMW, 6 - placebo. The treatment lasted for 7 days, the dosage was 200 - 400 ml 3 - 2 times daily. All patients were examined for proteinuria. The results are given in Tables 19 and 20.

### Example 7

Patient A.D. was admitted with complaints of weakness, dry mouth, heightened appetite, high level of glycolised fibrinogen in blood, proteinuria. After taking AMW for 7 days the level of glycolysed fibrinogen in blood was normalized, dropping from 8.20 to 6.40 nm/mg. There was no protein in urine any more.

The effectiveness of AMW in diabetes mellitus was demonstrated, the placebo did not yield comparable results.

### Immunodeficiency

6 patients were examined in an open experiment. Before and at 1.5, 47 and 191 hours after one day-long treatment with AMW the following data were collected: numbers of CD₃ - all mature T-lymphocites, CD₄ - T-helpers, inductors, CD₈ - T-suppressors, NK and β-lymphocites. The results are given in Table 21.

### Example 8

Patient, diagnosed as immunodeficient after glomerulonephritis, was treated with AMW. 90 minutes after intake the blood analysis revealed the increase of CD₃ - 2.6 times; CD₄ - 3.9 times; CD₈ - 3.1 times, NK - 3.5 times.

The effectiveness of AMW in immunodeficient state was demonstrated.

### Practically healthy persons from contaminated locality

10 persons were examined in a blind test. The level of Cs-137 in the daily urine was determined on a control day without intake of any curative liquid, after intake of placebo, after a day-long intake of AMW and after 10 days of AMW intake. The results are given in Table 22.

### Example 9

Patient No 1. The level of Cs-137 in the urine before treatment was 148.50 Bq/kg. After a placebo the level was unchanged. After a day-long intake of AMW (3x200 mL) the Cs-137 level reached 200.48 Bq/kg, an increase of 35% over the starting level. After 10 days of AMW treatment the level of CS-137 in the urine was 58.14 Bq/kg, or 60% less than the starting level. These results show the increased excretion of Cs-137 from the body.

### Influence on practically healthy persons

18 practically healthy persons were examined in an open experiment. The AMW was used in 100, 200 and 400 ml large doses. The influence of each dose was examined 24 and 72 hours after intake. No toxic effects were observed from 100 - 400 ml doses of AMW 24 or 72 hours after intake. The effects were within normal range, for example, the normal level of uric acid in urine is 200 - 700 mmol/l. After intake of AMW the level approached the lower limit. All patients had increased Cl⁻ level. After treatment with AMW the Cl⁻ level approached the lower limit. The Na⁺ level in blood of all patients sunk, approaching the lower limit.

### Example 10

A practically healthy patient before the experiment had slightly increased arterial pressure - 150/85 mm Hg. Anamnesis registered a family history of hypertonic condition. After treatment with AMW the blood pressure was normalized at 118/68 mm Hg.

Thus AMW was shown to be effective for practically healthy persons. At the same time AMW did not induce the drift of essential organic parameters out of normal range, thus confirming the absence of toxic effects.

**Table 1**

| Influence of placebo on protein level in daily urine Patients with glomerulonephritis | | | | |
|---|---|---|---|---|
| Nº | Patient | Protein in urine g/l | | Changes in % |
| | | before placebo | after placebo | |
| 1 | K.A. | 1.57 | 3.24 | +106% |
| 2 | T.A. | 1.50 | 1.20 | -20% |
| 3 | B.I. | 0.50 | 0.37 | -26% |
| 4 | B.E. | 0.02 | 0.02 | 0 |
| 5 | K.A. | 1.80 | 1.80 | 0 |
| 6 | U.J. | 0.20 | 0.20 | 0 |

**Table 2**

| Influence of AMW on protein level in daily urine Patients with glomerulonephritis | | | | |
|---|---|---|---|---|
| Nº | Patient | Protein in urine g/l | | Changes in % |
| | | before AMW intake | after AMW intake | |
| 1 | B.A. | 4.84 | 0.91 | -81% |
| 2 | K.I. | 4.84 | 0.07 | -99% |
| 3 | V.V. | 1.98 | 0.03 | -99% |
| 4 | G.A. | 2.94 | 2.52 | -14% |
| 5 | K.A. | 0.90 | 0.01 | -99% |
| 6 | B.I. | 2.52 | 0.12 | -95% |
| 7 | P.I. | 0.38 | 0 | -100% |
| 8 | R.D. | 0.02 | 0 | -100% |
| 9 | J.J. | 1.40 | 0.51 | -64% |
| 10 | S.G. | 0.36 | 0.003 | -99% |
| 11 | K.I. | 0.01 | 0.01 | 0% |
| 12 | B.N. | 0.56 | 0.44 | -22% |

**Table 3**

| Influence of single intake of AMW on protein level in daily urine Patients with glomerulonephritis 96 hours after intake | | | | | | |
|---|---|---|---|---|---|---|
| Patient | K⁺ level in blood plasma mmol/l | | Na⁺ level in blood plasma mmol/l | | Protein level in blood plasma g/l | |
| | before AMW | after AMW | before AMW | after AMW | before AMW | after AMW |
| 1 | 6.2 | 4.9 | 141 | 138 | 66 | 74 |
| 2 | 4.8 | 4.4 | 135 | 138 | 59 | 62 |
| 3 | 4.3 | 3.9 | 140 | 141 | 62 | 65 |
| 4 | 4.3 | 3.6 | 141 | 141 | 76 | 75 |
| 5 | 4.6 | 5.2 | 138 | 141 | 73 | 77 |
| 6 | 4.9 | 4.1 | - | - | 67 | 74 |

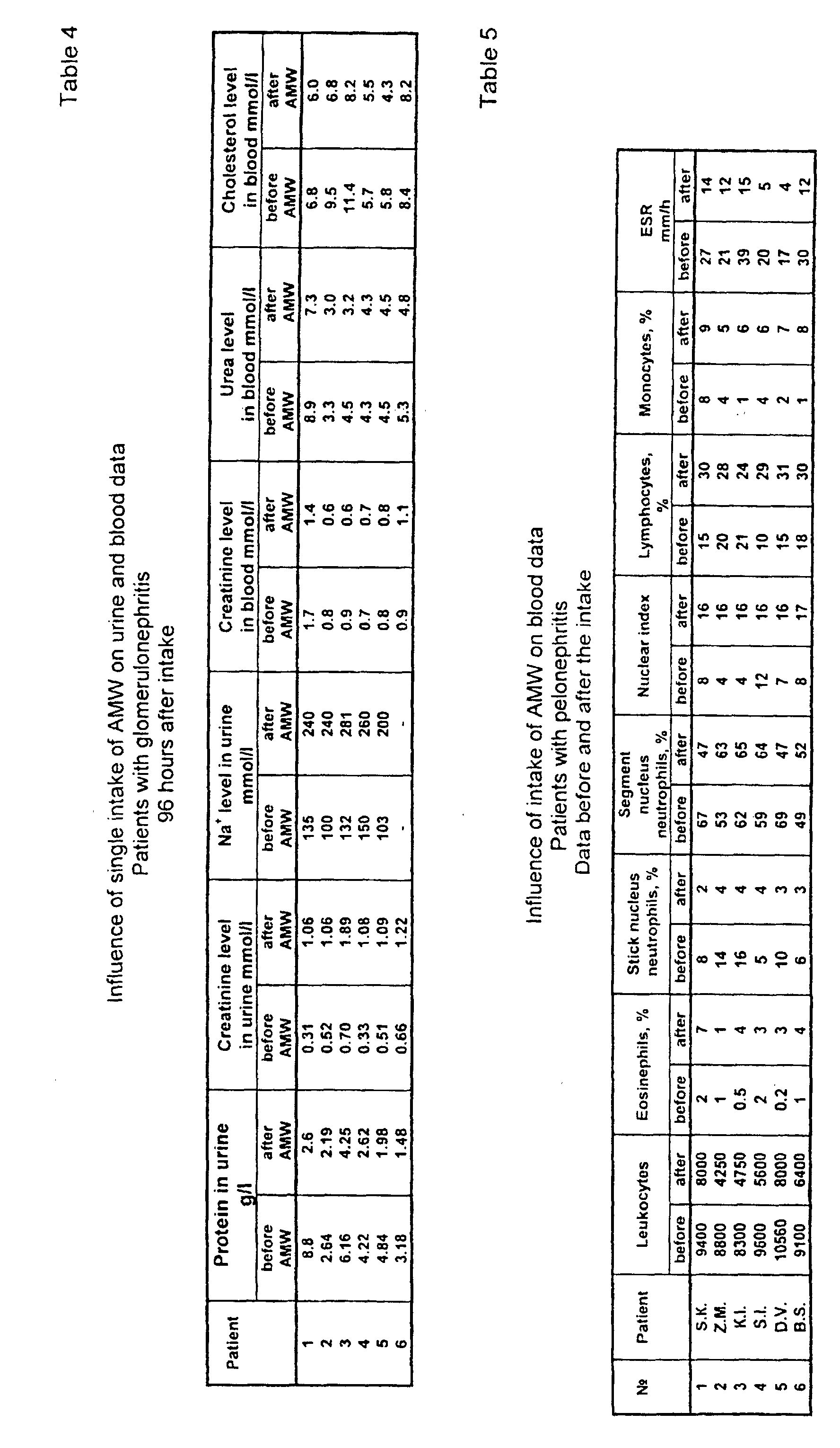

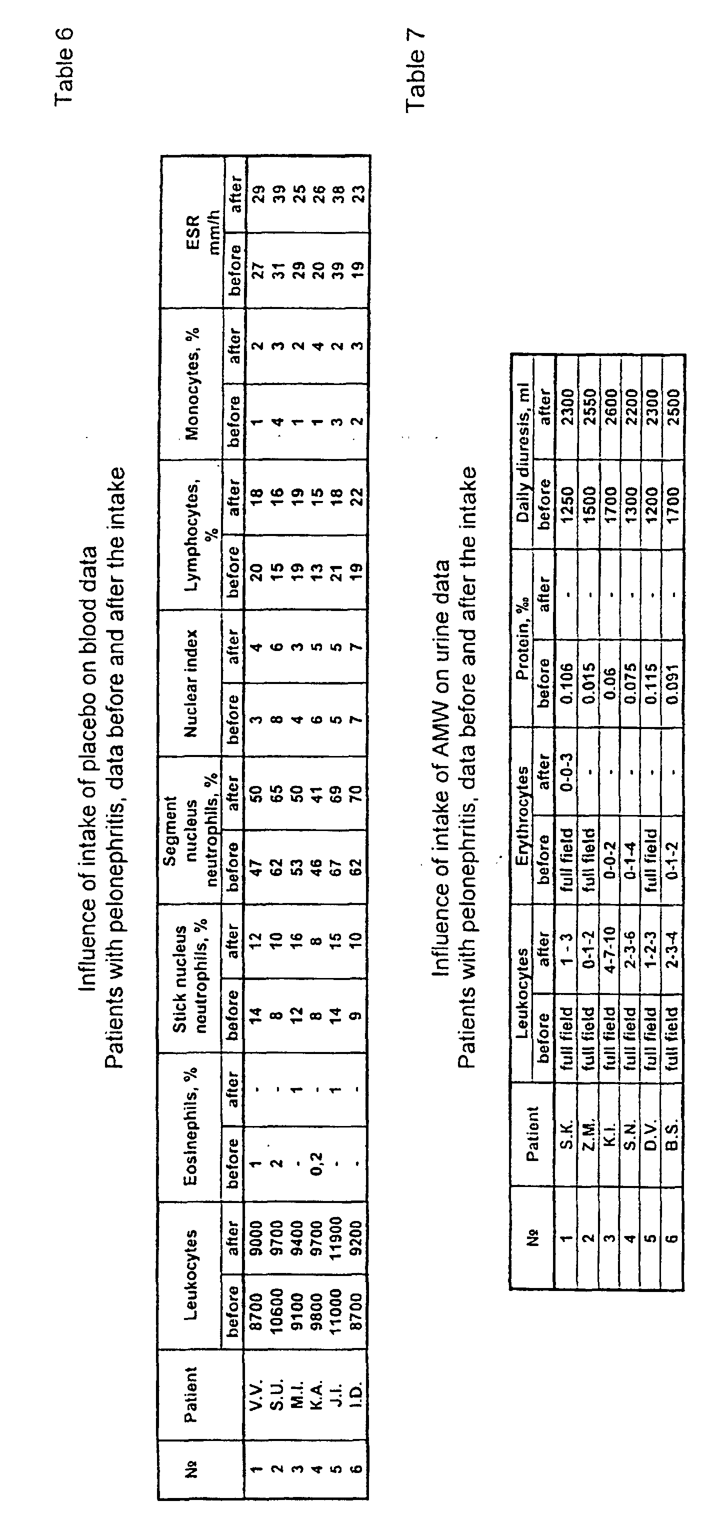

**Table 8**

| Influence of intake of placebo on urine data Patients with pelonephritis, data before and after the intake | | | | | | | | | |
|---|---|---|---|---|---|---|---|---|---|
| Nº | Patient | Leukocytes | | Erythrocytes | | Protein, ‰ | | Daily diuresis, ml | |
| | | before | after | before | after | before | after | before | after |
| 1 | V.V. | full field | full field | 1-2-4 | 1-2-2 | 0.090 | -0.096 | 1750 | 1600 |
| 2 | S.U. | full field | full field | rare | 0-0-3 | 0.075 | 0.072 | 1500 | 1600 |
| 3 | M.I. | full field | full field | 1-1-4 | 0-2-4 | 0.060 | 0.071 | 1350 | 1300 |
| 4 | K.A. | full field | full field | 0-1-2 | 1-1-3 | 0.100 | 0.106 | 12000 | 1800 |
| 5 | J.I. | full field | full field | full field | full field | 0.076 | 0.080 | 1700 | 1800 |
| 6 | I.D. | full field | full field | 0-2-2 | 1-1-4 | 0.02 | 0.02 | 1200 | 1250 |

**Table 9**

| Influence of intake of AMW on urine data Patients with pelonephritis, data before and after the intake | | | | | | | |
|---|---|---|---|---|---|---|---|
| Nº | Patient | Leukocytes | | Erythrocytes | | Protein, ‰ | |
| | | before | after | before | after | before | after |
| 1 | E.I. | full field | 30-40 | 20-40 | rare | 0.94 | - |
| 2 | R.A. | full field | - | 0-25 | - | 0.14 | 0.07 |
| 3 | Z.A. | full field | 20-25-30 | 40-50-60 | 10-5-10 | 0.35 | 0.14 |
| 4 | L.E. | full field | 20-30-35 | 20-25 | - | 0.76 | - |
| 5 | B.G. | full field | - | 30-35-40 | 1-1-10 | 0.24 | - |
| 6 | O.M. | full field | 1-1-3 | 10-10-20 | rare | 0.50 | - |

**Table 10**

| Influence of intake of placebo on urine data Patients with pelonephritis, data before and after the intake | | | | | | | |
|---|---|---|---|---|---|---|---|
| Nº | Patient | Leukocytes Erythrocytes | | | | Protein, ‰ | |
| | | before | after | before | after | before | after |
| 1 | N.J. | full field | full field | 12-10-10 | 10-10-15 | 2.74 | 2.00 |
| 2 | S.I. | full field | full field | rare | rare | 0.75 | 0.76 |
| 3 | M.T. | full field | full field | 24-20-50 | 30-35-30 | 0.02 | 0.05 |
| 4 | I.K. | full field | full field | 30-40-50 | 30-30-15 | 0.106 | 0.10 |
| 5 | S.M. | full field | full field | rare | 1-1-10 | 0.09 | 0.09 |
| 6 | G.T. | full field | full field | 3-2-20 | 2-2-20 | 0.19 | 0.20 |

**Table 11**

| Influence of AMW and placebo on total billirubin (mmol/l) in blood serum of virus A hepatite patients | | | | |
|---|---|---|---|---|
| Before and after AMW intake | | | | |
| Nº | Patient | Before | After | Changes in % |
| 1 | G.A. | 47 | 23 | -51% |
| 2 | K.N. | 47 | 18 | -62% |
| 3 | V.S. | 94 | 82 | -13% |
| 4 | G.G. | 158 | 95 | -40% |
| 5 | L.V | 158 | 35 | -78% |
| 6 | D.A. | 111 | 106 | -5% |

| Before and after intake of placebo | | | | |
|---|---|---|---|---|
| Nº | Patient | Before | After | Changes in % |
| 1 | A.U. | 82 | 82 | 0% |
| 2 | T.M. | 101 | 106 | +5% |
| 3 | B.S. | 94 | 97 | +3% |
| 4 | C.D. | 136 | 140 | +3% |
| 5 | D.J. | 54 | 54 | 0% |
| 6 | E.T. | 82 | 90 | +10% |

**Table 12**

| Influence of AMW and placebo on AlanineAT (units/l) in blood serum of virus A hepatite patients | | | | |
|---|---|---|---|---|
| Before and after intake of AMW | | | | |
| Nº | Patient | Before | After | Changes in % |
| 1 | G.A. | 1260 | 560 | 2x< |
| 2 | K.N. | 807 | 218 | 4x< |
| 3 | V.S. | 1658 | 654 | 2,5x< |
| 4 | G.G. | 2214 | 534 | 4x< |
| 5 | L.V. | 542 | 140 | 4x< |
| 6 | D.A. | 2962 | 972 | 3x< |

| Before and after intake of placebo | | | | |
|---|---|---|---|---|
| Nº | Patient | Before | After | Changes in % |
| 1 | A.U. | 1420 | 1558 | no |
| 2 | T.M. | 964 | 932 | no |
| 3 | B.S. | 1680 | 1720 | no |
| 4 | C.D. | 2062 | 2048 | no |
| 5 | D.J. | 1214 | 1433 | no |
| 6 | E.T. | 972 | 900 | no |

**Table 13**

| Changes of gallbladder diameter in a-p direction (mm) Practically healthy persons Intake of distilled water | | | | |
|---|---|---|---|---|
| Nº | Patient | Starting value | After 30 min | After 60 min |
| 1 | A.G. | 25 | 23 | 25 |
| 2 | T.G. | 16 | 15 | 15 |
| 3 | L.P. | 28 | 27 | 30 |
| 4 | I.P. | 23 | 24 | 23 |
| 5 | R.R. | 22 | 22 | 22 |
| 6 | D.S. | 24 | 25 | 22 |
| Mean value | | 23 | 23 | 23 |

**Table 14**

| Changes of gallbladder diameter in a-p direction (mm) Practically healthy persons Intake of saline (0.9% NaCI) | | | | | |
|---|---|---|---|---|---|
| Nº | Patient | Starting value | After 30 min | After 45 min | After 60 min |
| 1 | A.G. | 29 | 28 | 28 | 29 |
| 2 | T.G. | 19 | 18 | 17 | 18 |
| 3 | L.P. | 30 | 30 | 31 | 30 |
| 4 | I.P. | 30 | 31 | 31 | 32 |
| 5 | R.R. | 25 | 24 | 24 | 25 |
| 6 | D.S. | 26 | 26 | 26 | 25 |
| Mean value | | 26 | 26 | 26 | 26 |

**Table 15**

| Changes of gallbladder diameter in a-p direction (mm) Practically healthy persons Intake of AMW | | | | | | |
|---|---|---|---|---|---|---|
| Nº | Patient | Starting value | After 30 min | After 45 min | After 60 min | After 75 min |
| 1 | A.G. | 26 | 18 | 14 | 19 | 19 |
| 2 | T.G. | 26 | 18 | 17 | 17 | 19 |
| 3 | L.P. | 30 | 28 | 26 | 31 | 35 |
| 4 | I.P. | 29 | 26 | 30 | 28 | 28 |
| 5 | R.R. | 22 | 18 | 15 | 22 | 22 |
| 6 | D.S. | 28 | 24 | 22 | 27 | 27 |
| Mean value | | 27 | 22 | 21 | 24 | 25 |

**Table 16**

| Changes of gallbladder diameter in a-p direction (mm) Cholecystite patients Intake of AMW | | | | | | |
|---|---|---|---|---|---|---|
| Time after intake | Patient | | | | | |
| | Z.I. | A.I. | B.B. | L.I. | S.Z. | V.I. |
| 0 min | 26 | 28 | 28 | 35 | 28 | 37 |
| 30 min | 24 | 26 | 24 | 30 | 24 | 30 |
| 45 min | 20 | 29 | 26 | 30 | 28 | 28 |
| 60 min | 24 | 28 | 26 | 29 | 27 | 29 |
| 75 min | 28 | 26 | 30 | 31 | 27 | 32 |

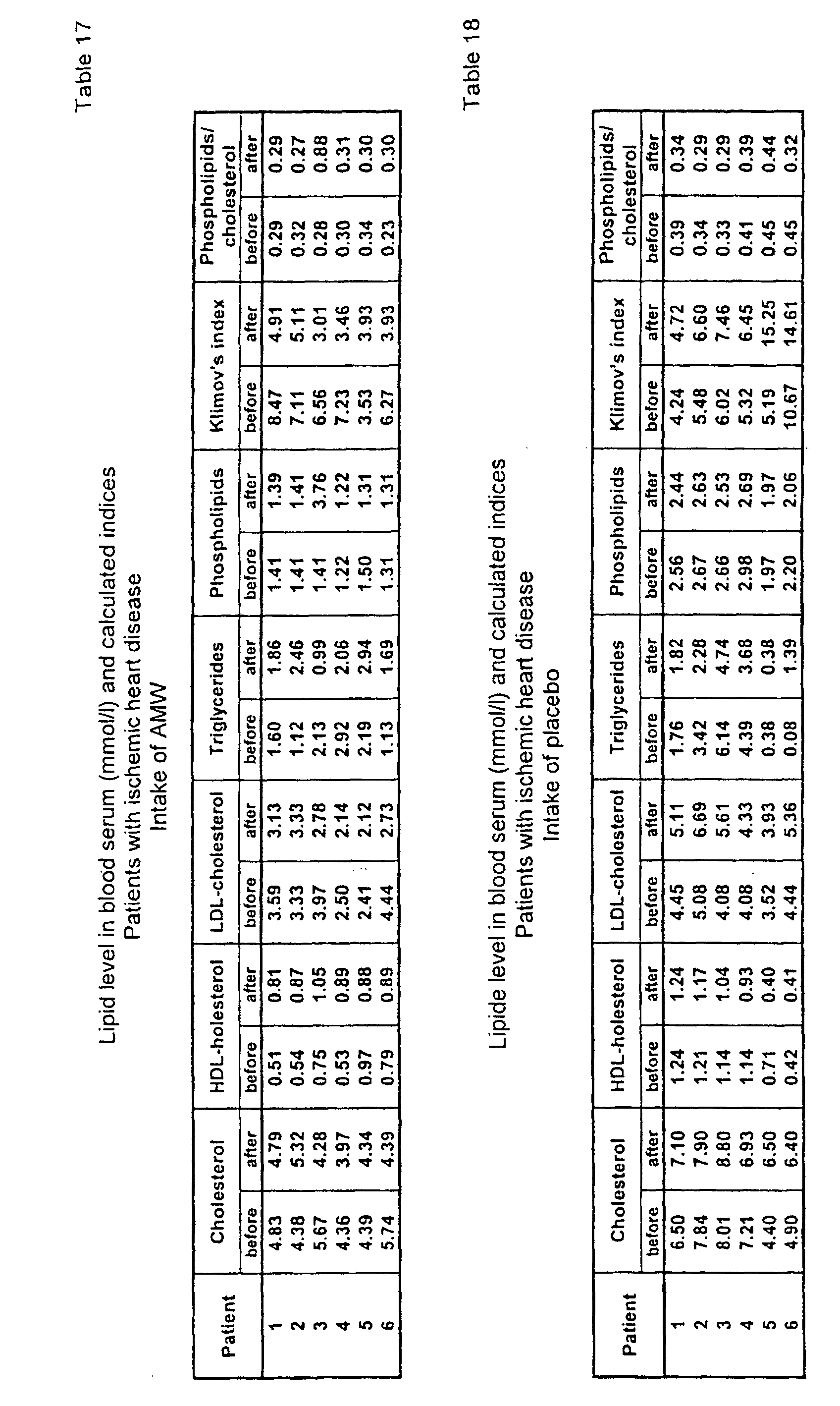

**Table 19**

| Influence of AMW and placebo on glycolysed fibrinogen level (nm/mg) in blood of patients with diabetes mellitus insuline therapy unaltered (Normal value range 5.4 - 7.2) | | | |
|---|---|---|---|
| Intake of AMW | | | |
| Patient | Before | After | Changes in % |
| 1 | 8.20 | 6.40 | -22 |
| 2 | 8.10 | 6.00 | -26 |
| 3 | 8.50 | 6.24 | -27 |
| 4 | 9.01 | 6.23 | -31 |
| 5 | 7.95 | 5.40 | -32 |
| 6 | 8.11 | 6.62 | -18 |

| Intake of placebo | | | |
|---|---|---|---|
| Patient | Before | After | Changes in % |
| 1 | 8.20 | 8.22 | no |
| 2 | 8.57 | 8.59 | no |
| 3 | 8.40 | 8.39 | no |
| 4 | 8.90 | 8.88 | no |
| 5 | 7.49 | 7.55 | no |
| 6 | 8.17 | 8.20 | no |

**Table 20**

| Influence of AMW and placebo on protein level (‰) in urine of patients with diabetes mellitus | | | |
|---|---|---|---|
| Intake of AMW | | | |
| Patient | Before | After | Changes in % |
| 1 | 0.46 | - | -100 |
| 2 | 0.33 | - | -100 |
| 3 | 2.50 | 0.03 | -98 |
| 4 | 2.47 | 1.00 | -60 |
| 5 | 2.80 | 0.99 | -65 |
| 6 | 2.43 | 0.07 | -98 |

| Intake of placebo | | | |
|---|---|---|---|
| Patient | Before | After | Changes in % |
| 1 | 3.33 | 3.00 | no |
| 2 | 0.015 | 0.046 | no |
| 3 | 0.396 | 0.462 | no |
| 4 | 2.20 | 1.98 | no |
| 5 | 0.066 | 2.97 | no |
| 6 | 3.00 | 2.97 | no |

**Table 21**

| Influence of AMW on patients with chronic glomerulonephritis with diagnosed immunodeficiency | | | | | |
|---|---|---|---|---|---|
| CD₃ (normal level 60 - 90%) | | | | | |
| Patient | Before intake | After intake | | | Changes (times) |
| | | 1.5 h | 47 h | 191 h | |
| 1 | 19 | 51 | 45 | 50 | 2.6 |
| 2 | 30 | 80 | 85 | 80 | 2.7 |
| 3 | 23 | 60 | 65 | 65 | 2.8 |
| 4 | 15 | 59 | 55 | 55 | 3.8 |
| 5 | 40 | 80 | 80 | 80 | 2.0 |
| 6 | 18 | 50 | 50 | 50 | 2.8 |

| CD₄ (normal level 33-60%) | | | | | |
|---|---|---|---|---|---|
| Patient | Before intake | After intake | | | Changes (times) |
| | | 1.5 h | 47 h | 191 h | |
| 1 | 9 | 38 | 30 | 37 | 4.1 |
| 2 | 18 | 45 | 45 | 49 | 2.7 |
| 3 | 20 | 40 | 49 | 54 | 2.4 |
| 4 | 12 | 60 | 58 | 58 | 4.8 |
| 5 | 8 | 35 | 40 | 40 | 5.0 |
| 6 | 15 | 45 | 45 | 45 | 3.0 |

| CD₈ (normal level 20-32%) | | | | | |
|---|---|---|---|---|---|
| Patient | Before intake | After intake | | | Changes (times) |
| | | 1.5 h | 47 h | 191 h | |
| 1 | 8 | 21 | 25 | 29 | 3.6 |
| 2 | 5 | 30 | 30 | 30 | 6.0 |
| 3 | 10 | 25 | 28 | 32 | 3.2 |
| 4 | 7 | 20 | 30 | 30 | 4.3 |
| 5 | 5 | 28 | 28 | 28 | 5.6 |
| 6 | 11 | 30 | 28 | 28 | 2.5 |

| CD₂₅ (normal level 0-5%) | | | | | |
|---|---|---|---|---|---|
| Patient | Before intake | After intake | | | Changes (times) |
| | | 1.5 h | 47 h | 191 h | |
| 1 | 8 | 10 | 8 | 4 | 2.0 |
| 2 | 11 | 4 | 4 | 4 | 2.7 |
| 3 | 9 | 2 | 2 | 3 | 3.0 |
| 4 | 8 | 4 | 4 | 4 | 2.0 |
| 5 | 5 | 5 | 5 | 5 | - |
| 6 | 7 | - | 3 | 3 | 2.3 |

| NK (normal level 8-22%) | | | | | |
|---|---|---|---|---|---|
| Patient | Before intake | After intake | | | Changes (times) |
| | | 1.5 h | 47 h | 191 h | |
| 1 | 6 | 12 | 19 | 33 | 5.5 |
| 2 | 5 | 20 | 21 | 20 | 4.0 |
| 3 | 2 | 18 | 19 | 18 | 9.0 |
| 4 | 6 | 33 | 30 | 33 | 5.5 |
| 5 | - | - | - | - | - |
| 6 | - | - | - | - | - |

| β-lymphocytes (normal level 5-20%) | | | | | |
|---|---|---|---|---|---|
| Patient | Before intake | After intake | | | Changes (times) |
| | | 1.5 h | 47 h | 191 h | |
| 1 | 15 | 8 | 16 | 18 | 1.2 |
| 2 | 18 | 17 | 18 | 18 | - |
| 3 | 20 | 10 | 15 | 10 | 0.5 |
| 4 | 18 | 10 | 15 | 10 | 0.5 |
| 5 | 5 | 7 | 5 | 7 | 1.4 |
| 6 | 9 | 10 | 12 | 10 | 1.1 |

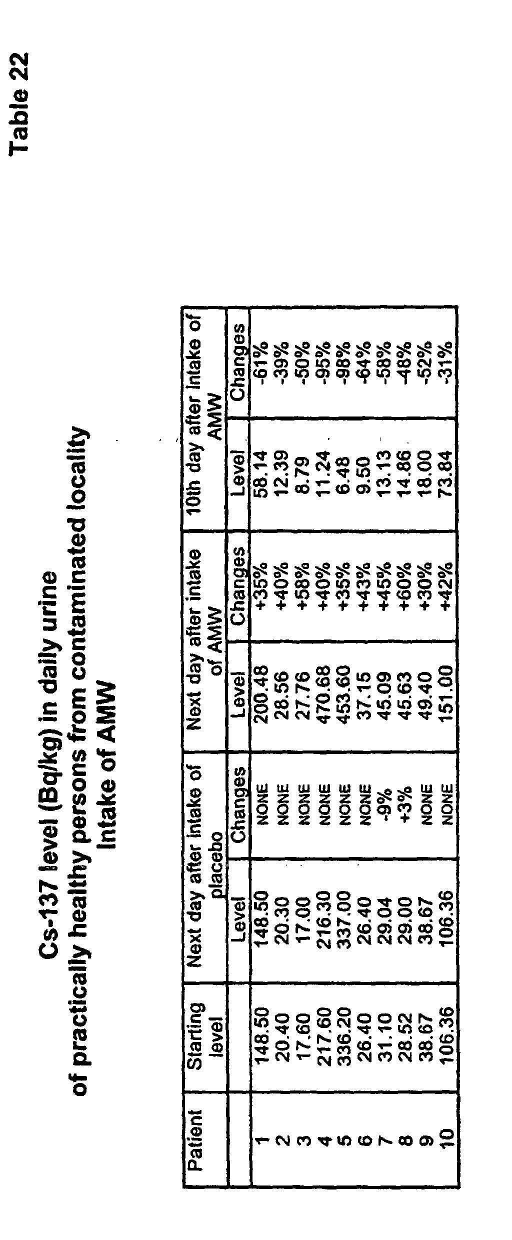

## Claims

1. An artificial medicinal-prophylactic water, ***characterized in that*** it contains the following components, in per cent weight:
| | |
|---|---|
| magnesium sulphate | 0.0005 - 0.01, |
| potassium chloride | 0.001 - 0.02, |
| tris-(hydroxymethyl)aminomethane | 0.005 - 0.02, |
| tris-(hydroxymethyl)aminomethane hydrochloride | 0.01 - 0.04, |
| distilled water | up to 100. |

2. A water according to claim 1 further including soft drink additives.

3. A water according to claim 1 or 2
a) for use in the treatment and prevention of any of the following conditions: disturbances of kidney and liver functions, gallbladder diskinesias, diabetes mellitus, disturbances of arterial pressure, atherosclerosis changes in blood vessels;
b) for the normalization of immunity factors of cells;
c) for the normalization of aberrations from homeostasis; or
d) for intensifying the excretion of radioactive metal ions, particularly Cs-137.

4. A water according to claim 1 or 2 for use in the manufacture of a pharmaceutical preparation for any of the uses recited in paragraphs a) - d) of claim 3.

5. A method of stabilizing a mineral water by the addition of 0.005-0.02 wt. percent tris-(hydroxymethyl)aminometharie and 0.01 - 0.04 wt. percent tris-(hydroxymethyl)aminomethane hydrochloride.

## Patentansprüche

1. Künstliches medizinisch-prophylaktisches Wasser, **dadurch gekennzeichnet, dass** es die folgenden Komponenten in Gew.-% enthält:
| | |
|---|---|
| Magnesiumsulfat | 0,0005 - 0,01 |
| Kaliumchlorid | 0,001 - 0,02 |
| Tris-(hydroxymethyl)aminomethan | 0,005 - 0,02 |
| Tris-(hydroxymethyl)aminomethanhydrochlorid | 0,01 -0,04 |
| destilliertes Wasser | ad 100. |

2. Wasser nach Anspruch 1, das außerdem Softdrink-Additive enthält.

3. Wasser nach Anspruch 1 oder 2
a) für die Verwendung bei der Behandlung und Prävention irgendeiner der folgenden Zustände: Störungen der Nieren- und Leberfunktionen, Gallenblasen-Dyskinesie, Diabetes mellitus, Störungen des arteriellen Blutdrucks, arteriosklerotische Veränderungen der Blutgefäße;
b) zur Normalisierung von Immunitätsfaktören der Zellen;
c) zur Normalisierung von Abweichungen von der Homöostase oder
d) zur Verstärkung der Exkretion von radioaktiven Metallionen, insbesondere Cs-137.

4. Wasser nach Anspruch 1 oder 2 für die Verwendung zur Herstellung eines pharmazeutischen Präparats für irgendeine der in den Abschnitten (a) bis (d) des Anspruchs 3 aufgezählten Verwendungen.

5. Verfahren zum Stabilisieren eines Mineralwassers durch Zugabe von 0,005 bis 0,02 Gew.-% Tris-(hydroxymethyl)aminomethan und 0,01 bis 0,04 Gew.-% Tris-(hydroxymethyl)aminomethanhydrochlorid.

## Revendications

1. Eau artificielle médico-prophylactique, **caractérisée en ce qu'**elle contient, en % en poids :
| | |
|---|---|
| sulfate de magnésium | 0,0005-0,01 |
| chlorure de potassium | 0,001-0,02 |
| tris-(hydroxyméthyl)aminométhane | 0,005-0,02 |
| tris-(hydroxyméthyl)aminométhane,HCI | 0,01-0,04 |
| eau distillée, jusqu'à | 100. |

2. Eau suivant la revendication 1, comprenant en outre des additifs pour boison non-alcoolisée.

3. Eau suivant la revendication 1 ou 2,
a) pour utilisation dans le traitement et la prévention de l'un quelconque des états suivants : perturbations des fonctions rénales et hépatiques, dyskinésies de la vésicule biliaire, diabète sucré, perturbations de la pression artérielle, modifications athérosclérosantes dans les vaisseaux sanguins ;
b) pour la normalisation des facteurs immunitaires des cellules ;
c) pour la normalisation des aberrations de l'homoeostasie ; ou
d) pour intensifier l'excrétion d'ions de métaux radioactifs, notamment le ¹³⁷Cs.

4. Eau suivant la revendication 1 ou 2, pour utilisation dans la fabrication d'une préparation pharmaceutique destinée à l'une quelconque des utilisations visées aux paragraphes a)-d) de la revendication 3.

5. Procédé pour stabiliser une eau minérale, par addition de 0,005-0,02 % en poids de tris-(hydroxyméthyl)aminométhane et de 0,01-0,04 % en poids de chlorhydrate de tris-(hydroxyméthyl)aminométhane.
